# DEMANDE DE BREVET EUROPEEN

(11) **EP 1 426 036 A1**
(43) Date de publication de la demande: **09.06.2004**
(21) Numéro de dépôt: 03292849.1
(22) Date de dépôt: 18.11.2003
(51) Int. Cl.: A61K 7/13

(54) **Utilisation de pigments latents pour la coloration haute tenue, composition contenant lesdits pigments et procédés les mettant en oeuvre**

(30) Priorité: 20.11.2002 FR 0214535
(71) Demandeur: L'OREAL, 75008 Paris (FR)
(72) Inventeur: Greaves, Andrew, 77144 Montevrain (FR); Kravtchenko, Sylvain, 92600 Asnieres (FR); Lagrange, Alain, 77700 Coupvray (FR)
(74) Mandataire: Casalonga, Axel

(57) **Abrégé**

L'invention concerne l'utilisation pour la coloration de fibres kératiniques d'au moins un pigment latent, soluble dans un milieu approprié pour la teinture, susceptible d'être transformé dans les fibres en pigment insoluble dans l'eau par voie chimique, thermique ou photochimique, de formule

A(B)ₓ

avec A représentant le radical chromophore de colorants,
et B un atome d'hydrogène ou un groupe de formule (II), 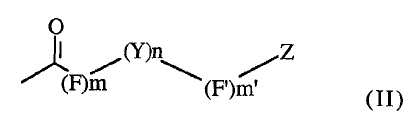 avec Z représentant un groupement hydrosolubilisant cationique Z⁺ ou un résidu de polyéthylèneglycol, Y un hétéroatome, F et F' une chaîne alkylène en C₁-C₁₄ linéaire ou ramifiée, pouvant contenir des hétéroatomes et pouvant être substituée par un ou plusieurs groupements hydroxy, amino, halogène.

L'invention concerne également les procédés de coloration des fibres kératiniques utilisant les pigment latents, des compositions particulières les contenant ainsi que des dispositifs de teinture ou « kits » à plusieurs compartiments.

## Description

L'invention a pour objet l'utilisation des pigments latents pour la coloration des fibres kératiniques, de préférence des fibres kératiniques humaines, et en particulier des cheveux, des procédés de teinture des fibres kératiniques humaines mettant en oeuvre ces composés et des compositions particulières contenant ces composés.

Il est connu de teindre les fibres kératiniques humaines et en particulier les cheveux avec des compositions tinctoriales contenant des précurseurs de colorant d'oxydation, appelés généralement bases d'oxydation. Ces bases d'oxydation sont des composés incolores ou faiblement colorés qui, associés à des produits oxydants, donnent naissance par un processus de condensation oxydative à des composés colorés.

On sait également que l'on peut faire varier les nuances obtenues avec ces bases d'oxydation en les associant à des coupleurs ou modificateurs de coloration. La variété des molécules mises en jeu au niveau des bases d'oxydation et des coupleurs permet l'obtention d'une riche palette de couleurs.

Ce procédé de coloration d'oxydation consiste à appliquer sur les fibres kératiniques, des bases d'oxydation ou un mélange de bases d'oxydation et de coupleurs avec un agent oxydant, par exemple de l'eau oxygénée, à laisser poser, puis à rincer les fibres. Les colorations qui en résultent sont permanentes, puissantes, et résistantes aux agents extérieurs, notamment à la lumière, aux intempéries, aux lavages, à la transpiration et aux frottements.

Si ces colorations permettent d'obtenir des reflets intenses et tenaces, l'utilisation d'un agent oxydant tel que l'eau oxygénée, souvent en présence d'un agent alcalin, entraîne une dégradation de la fibre kératinique.

Il existe donc un réel besoin de rechercher des colorants chromatiques qui permettent de teindre les fibres kératiniques humaines aussi puissamment que les colorants d'oxydation, qui soient aussi stables qu'eux à la lumière, soient également résistants aux intempéries, aux lavages et à la transpiration. Il existe aussi un réel besoin de rechercher des colorants qui permettent d'obtenir des montées de couleur comparables à celles obtenues avec les précurseurs de colorants d'oxydation. De plus, la demanderesse a cherché à obtenir des colorants présentant une bonne innocuité et ne dégradant pas la fibre kératinique.

Les inventeurs ont découvert que l'utilisation de pigments latents permettait de colorer intensément et très durablement la fibre kératinique sans pour autant la dégrader. Ces performances étonnantes sont obtenues à l'aide de pigments latents qui sont des molécules solubles dans les formulations de coloration et qui conduisent à des molécules très peu solubles voire insolubles dans l'eau lorsqu'elles sont dans les fibres kératiniques.

Les pigments latents sont des substances connues. Ils ont été décrits dans la demande de brevet WO 98/32802 notamment dans le domaine des peintures, des encres ou des plastiques.

Un pigment latent est un composé insoluble, ou très peu soluble dans l'eau (comme par exemple un pigment), qui a été transformé de façon à le rendre soluble dans une formulation aqueuse. Ainsi la formulation aqueuse de coloration contenant le pigment latent est appliquée sur les fibres kératiniques. Après diffusion de ce composé soluble dans les fibres, on fait subir une réaction de "cassure" conduisant à la formation d'une molécule, le pigment final, substantiellement insoluble. Ces réactions de "cassure" sont des méthodes connues. Ce sont des réactions thermiques, chimiques ou photochimiques sur le pigment latent, qui est soluble. Ces réactions provoquent la rupture de la liaison entre le radical chromophore et le ou les groupes solubilisants. Cette réaction de rupture de liaison peut être considérée comme une réaction de régénération du composé d'origine peu soluble et peut être réalisée à l'intérieur de la fibre kératinique.

La présente invention a pour objet l'utilisation pour la coloration de fibres kératiniques, en particulier de fibres kératiniques humaines, plus particulièrement les cheveux, de pigments latents. L'invention concerne également une composition cosmétique comprenant au moins un pigment latent. L'invention a pour autre objet des procédés de coloration des fibres kératiniques utilisant les pigments latents ainsi que des dispositifs de teinture ou « kits » à plusieurs compartiments.

D'autres caractéristiques, aspects, objets et avantages de l'invention figurant dans la description ci-dessous permettront de mieux cerner l'invention.

Conformément à l'invention, on utilise pour la coloration de fibres kératiniques, en particulier de fibres kératiniques humaines, plus particulièrement les cheveux, des pigments latents, solubles dans un milieu approprié pour la teinture, susceptible d'être transformés dans les fibres en pigments insolubles dans l'eau par voie chimique, thermique ou photochimique.

Le pigment latent utilisé selon la présente invention est de préférence représenté par la formule (I)

A(B)ₓ (I)

dans laquelle x représente un nombre entier allant de 1 à 8,
A représente le radical chromophore de colorants comprenant un hétéroatome choisi parmi N, O et S, et
- lorsque x=1, B représente un groupe de formule (II),
- lorsque x est supérieur à 1, B désigne un atome d'hydrogène ou un groupe de formule (II),
B désignant au moins une fois un groupe de formule (II),

Le groupe de formule (II) correspond à 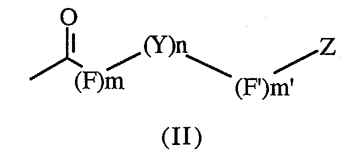 dans laquelle
Z représente un groupement hydrosolubilisant cationique Z⁺ ou un résidu de polyéthylèneglycol,
Y représente un hétéroatome choisi dans le groupe formé par N, O, et S, Y étant de préférence O,
F et F' représentent, indépendamment l'un de l'autre, une chaîne alkylène en C₁-C₁₄ linéaire ou ramifiée, pouvant contenir des hétéroatomes et pouvant être substituée par un ou plusieurs groupements hydroxy, amino, halogène,
n, m, m' désignent indépendamment les uns des autres, 0 ou 1,
B étant lié à un hétéroatome choisi dans le groupe N, O, et S du chromophore A.

Z⁺ est de préférence un groupe aliphatique, un groupe aromatique, un groupe carbocyclique saturé ou non, un groupe hétérocyclique et porte au moins un atome d'azote quaternisé.

De préférence, le chromophore A est le radical de colorants de type pérylène, quinacridone, dioxazine, isoindoline, indigo, bisisoindoline, phtalocyanine, pyrrolo-pyrrole, quinophtalone, azo, anthraquinone, indanthrone, isoindolinone, naphtoquinone, benzoquinone, azométhine.

On entend par "halogène", un élément choisi parmi le fluor, le chlore, le brome et l'iode.

Plus particulièrement, le radical chromophore A est choisi parmi :
- les dérivés du pérylène de formules (III) ou (IV) 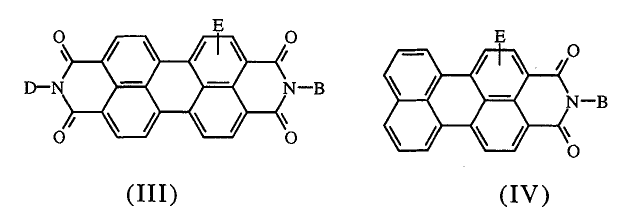 dans lesquelles D représente un atome d'hydrogène, un atome d'halogène, un groupe alkyle en C₁-C₂₄ linéaire ou ramifié, de préférence en C₁-C₆, ou un groupe phényle, benzyle, phénéthyle substitués ou non par un groupe alkyle en C₁-C₆, ou un groupe de formule B,
   E représente un atome d'hydrogène, un atome d'halogène, un groupe alkyle en C₁-C₂₄ linéaire ou ramifié, de préférence en C₁-C₆, un groupe alcoxy en C₁-C₆ ou un groupe phényle,
- les quinacridones de formule (V) 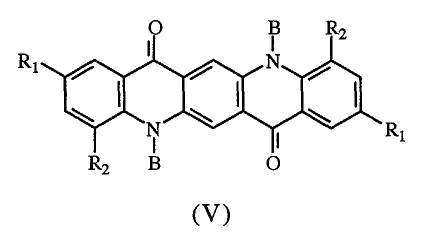 dans lesquelles R₁ et R₂, indépendamment l'un de l'autre, représentent un atome d'hydrogène, un atome d'halogène, un groupe alkyle en C₁-C₂₄ linéaire ou ramifié, de préférence en C₁-C₆, un groupe alcoxy en C₁-C₆ ou un groupe phényle,
- les dioxazines de formules (VI) ou (VII) 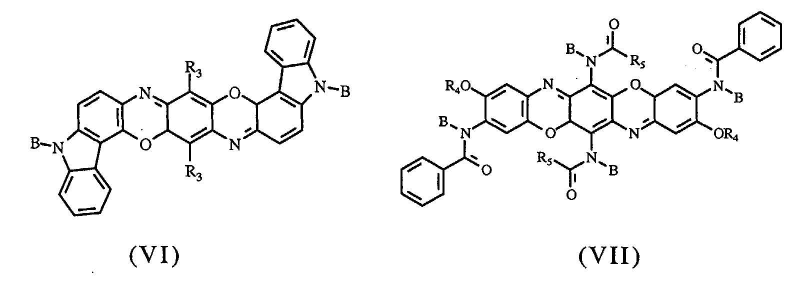 dans lesquelles R₃ représente un atome d'hydrogène, un atome d'halogène ou un groupe alkyle en C₁-C₂₄ linéaire ou ramifié, de préférence en C₁-C₆,
   R₄ et R₅ représentent chacun un groupe alkyle en C₁-C₄,
- les isoindolines de formules (VIII), (IX) ou (X) 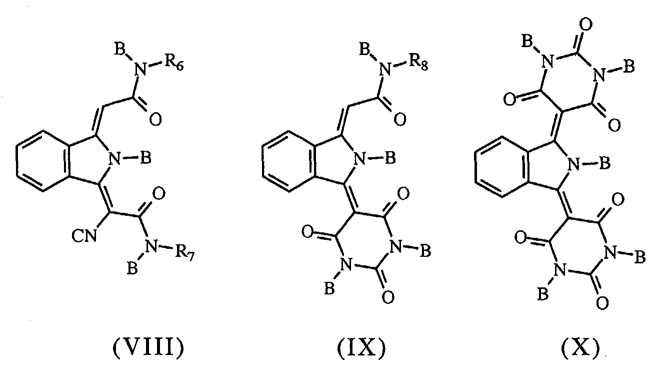 dans lesquelles R₆ est représenté par la formule (XI),
   R₇ représente un atome d'hydrogène, un groupe alkyle en C₁-C₂₄ linéaire ou ramifié, de préférence en C₁-C₆, un groupe benzyle ou un groupe de formule (XII),
   R₈ représente un atome d'hydrogène, le groupe de formule (XI)
   ou le groupe B, 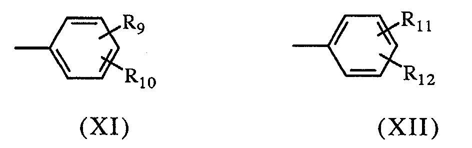 R₉, R₁₀, R₁₁ et R₁₂, indépendamment les uns des autres, représentent un atome d'hydrogène, un atome d'halogène, un groupe alkyle en C₁-C₂₄ linéaire ou ramifié, de préférence en C₁-C₆, un groupe alkoxy en C₁-C₆ ou un groupe trifluorométhyle,
- les dérivés d'indigo de formule (XIII) 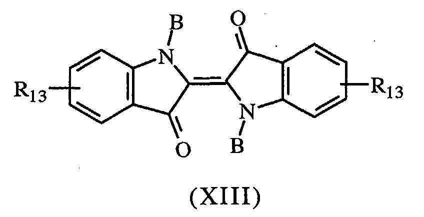 dans laquelle R₁₃ représente un atome d'hydrogène, un atome d'halogène, un groupe alkyle en C₁-C₆ linéaire ou ramifié, un groupe alkoxy en C₁-C₆ ou un groupe nitrile,
- les dérivés de bisindolinones de formules (XIV) ou (XV) 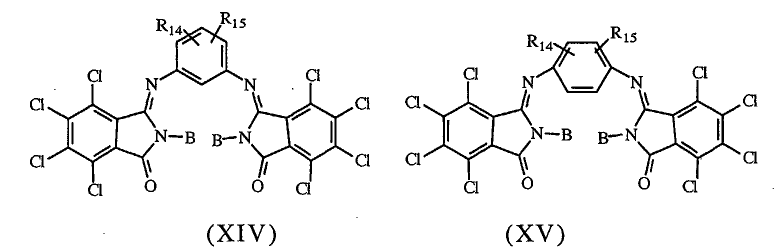 dans lesquelles R₁₄ et R₁₅, indépendamment l'un de l'autre, représentent un atome d'hydrogène, un atome d'halogène, un groupe alkyle en C₁-C₄ linéaire ou ramifié,
- les dérivés d'anthraquinoïdes de formules (XVI) ou (XVII) 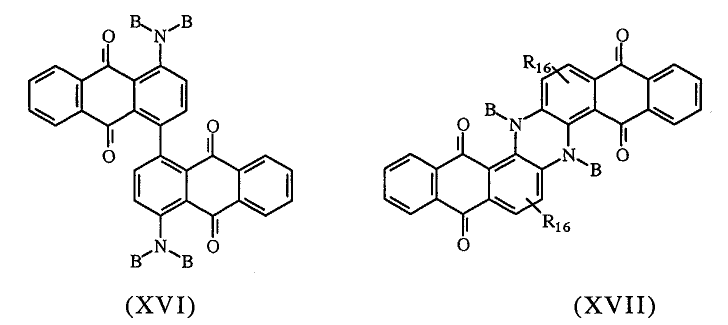 dans lesquelles R₁₆ représente un atome d'hydrogène ou un atome d'halogène,
- les dérivés de phthalocyanine de formule (XVIII) 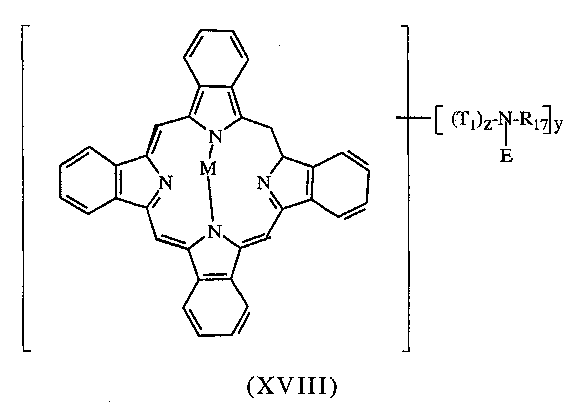 dans laquelle M représente H₂, un métal divalent choisi parmi le cuivre, le magnésium, le fer, le zinc, l'aluminium, le manganèse, le calcium, le baryum ou un groupe métallique divalent tel que MnO ou TiO,
   T₁ représente le groupe -CHR₁₈-, -CO-, -SO₂-,
   R₁₇ représente un atome d'hydrogène, un groupe alkyle en C₁-C₆ linéaire ou ramifié, un groupe -N(B)R₁₈, -N(B)₂, -NHCOR₁₉, -COR₁₉,
   ou le groupe de formule 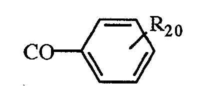 R₁₈ représente un atome d'hydrogène, un groupe alkyle en C₁-C₆ linéaire ou ramifié,
   R₁₉ représente un groupe alkyle en C₁-C₆ linéaire ou ramifié,
   R₂₀ représente un atome d'hydrogène, un atome d'halogène, un groupe alkyle en C₁-C₆ linéaire ou ramifié, un groupe alkoxy en C₁-C₆,
   z représente 0 ou 1,
   y représente un nombre entier de 1 à 8,
- les dérivés de pyrrolo-pyrroles de formules (XIX) ou (XX) 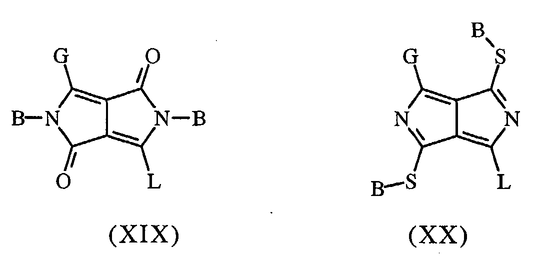 dans lesquels G et L, indépendamment l'un de l'autre, peuvent avoir comme signification : 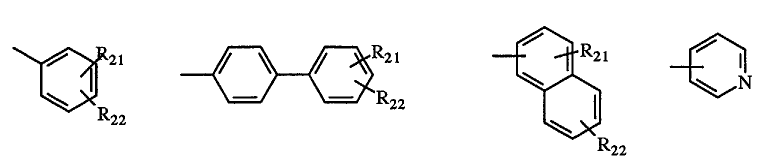 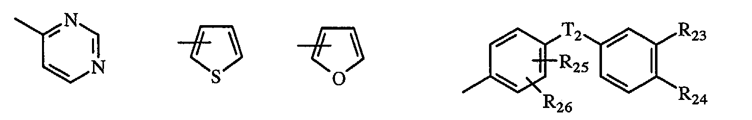 R₂₁ et R₂₂, indépendamment l'un de l'autre, représentent un atome d'hydrogène, un atome d'halogène, un groupe alkyle en C₁-C₂₄ linéaire ou ramifié, de préférence en C₁-C₆, un groupe alkoxy en C₁-C₆, un groupe alkylthio en C₁-C₁₈, un groupe alkylamino en C₁-C₁₈, un groupe cyano, nitro, phényle, trifluorométhyle, cycloalkyle en C₅-C₆, un groupe -C=N-(alkyle C₁-C₂₄ de préférence en C₁-C₆), un groupe de formule 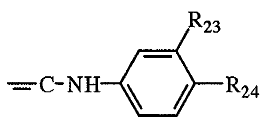 un groupe imidazolyle, pyrazolyle, triazolyle, pipérazinyle, pyrrolyle, oxazolyle, benzoxazolyle, benzothiazolyle, benzimidazolyle, morpholinyle, pipéridinyle ou pyrrolidinyle,
   T₂ représente -CH₂-, -CH(CH₃)-, -C(CH₃)₂-, -CH=N-, -N=N-, -O-, -S-, -SO-, -SO₂- ou -NHR₂₇,
   R₂₃ et R₂₄, indépendamment l'un de l'autre, représentent un atome d'hydrogène, un halogène, un groupe alkyle en C₁-C₆ linéaire ou ramifié, un groupe alkoxy en C₁-C₆ ou -CN,
   R₂₅ et R₂₆, indépendamment l'un de l'autre, représentent un atome d'hydrogène, un atome d'halogène, un groupe alkyle en C₁-C₆ linéaire ou ramifié,
   R₂₇ représente un atome d'hydrogène ou un groupe alkyle en C₁-C₆ linéaire ou ramifié,
- les dérivés de quinophthalones de formules (XXI) ou (XXII) 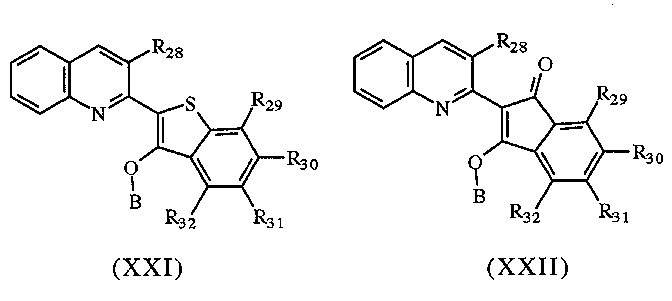 dans lesquelles R₂₈ représente un atome d'hydrogène ou un groupe OB,
   R₂₉, R₃₀, R₃₁ et R₃₂, indépendamment les uns des autres, représentent un atome d'hydrogène, un atome d'halogène, un groupe COOC₁-C₆alkyle linéaire ou ramifié, ou un groupe CONHC₁-C₆alkyle linéaire ou ramifié,
- les composés azoïques de formules de (XXIII) à (XXVIII) 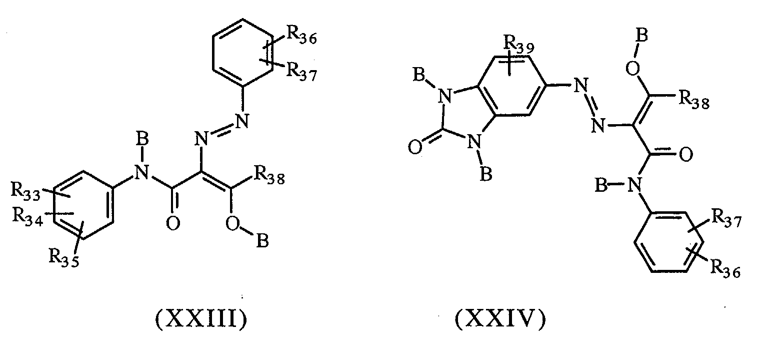 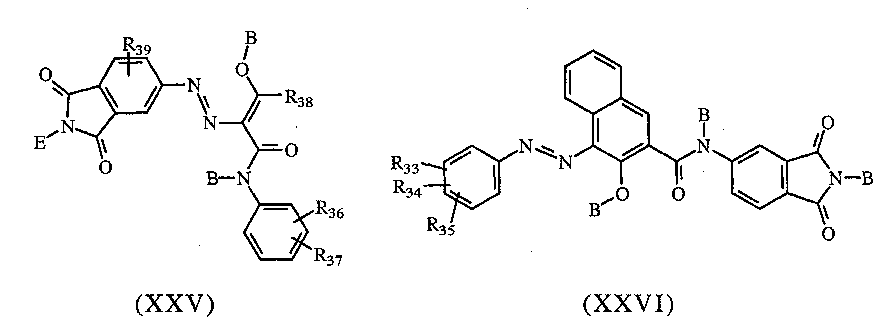 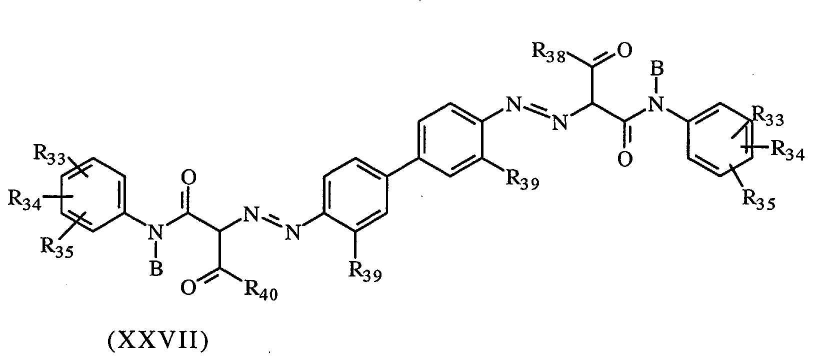 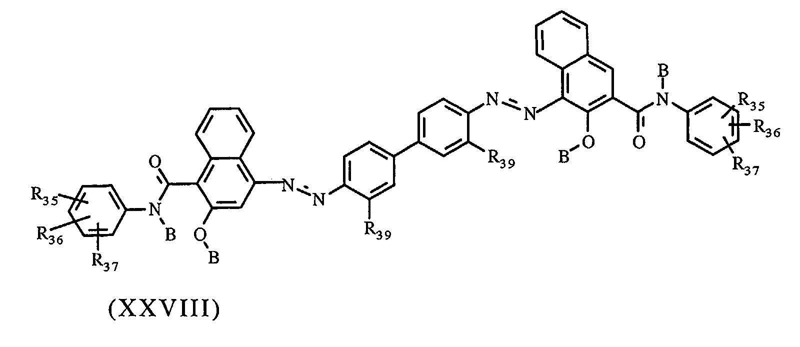 dans lesquelles R₃₃, R₃₄, R₃₅, R₃₆, R₃₇, R₃₈ et R₄₀ indépendamment les uns des autres, représentent chacun un atome d'hydrogène, un atome d'halogène, un groupe alkyle en C₁-C₆ linéaire ou ramifié, un groupe alkoxy en C₁-C₆ , un groupe nitro, acétyle ou un groupe SO₂NHC₁-C₆alkyle,
   R₃₉ représente un atome d'hydrogène, un atome d'halogène, un groupe alkyle en C₁-C₆ linéaire ou ramifié, un groupe alkoxy en C₁-C₆,
- les dérivés d'anthraquinones de formule (XXIX) ou (XXX) 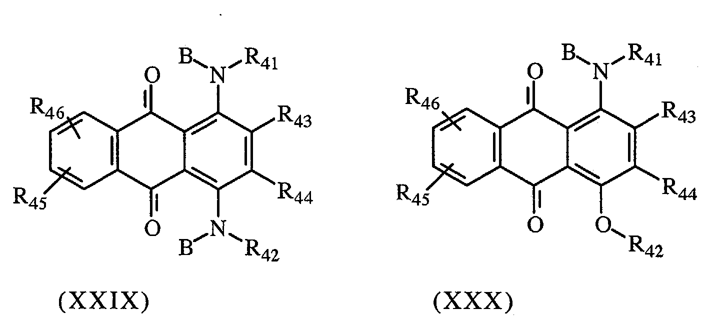 dans lesquelles R₄₁ et R₄₂, indépendamment l'un de l'autre, représentent un atome d'hydrogène, un atome d'halogène, un groupe alkyle en C₁-C₁₂ linéaire ou ramifié, un groupe alkoxy en C₁-C₆, ou un groupe aryle en C₆-C₁₂ qui est non substitué ou substitué par un ou plusieurs atomes d'halogène, un ou plusieurs groupes alkyle en C₁-C₆ linéaires ou ramifiés, nitro, acétyle ou groupe SO₂NHC₁-C₆alkyle ou SO₂NH₂,
   R₄₃ et R₄₄, indépendamment l'un de l'autre, représentent un atome d'hydrogène, un atome d'halogène, un groupe alkyle en C₁-C₆ linéaire ou ramifié, un groupe alkoxy en C₁-C₆ , un groupe nitro, cyano, CONH₂, SO₂NHC₁-C₆ alkyle, SO₂NH₂, SO₃H, SO₃Na ou un groupe aryle en C₆-C₁₂ qui est non substitué ou substitué par un ou plusieurs atomes d'halogène, un ou plusieurs groupes alkyle en C₁-C₆ linéaires ou ramifiés, nitro, acétyle ou SO₂NHC₁-C₆alkyle ou par SO₂NH₂,
   R₄₅ et R₄₆ représentent indépendamment l'un de l'autre, un atome d'hydrogène, un atome d'halogène, un groupe alkoxy en C₁-C₆, un groupe nitro, cyano, hydroxyle ou amino,
- les dérivés d'azométhine de formule (XXXI) 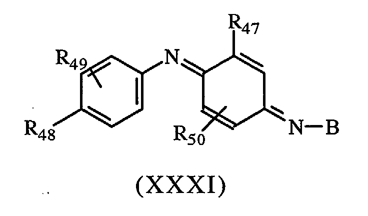 R₄₇, R₄₈, R₄₉ et R₅₀, indépendamment les uns des autres, représentent un atome d'hydrogène, un atome d'halogène, un groupe alkyle en C₁-C₆ linéaire ou ramifié, un groupe alkoxy en C₁-C₆ , un groupe nitro, cyano, CONH₂, SO₂NHC₁-C₆ alkyle, SO₂NH₂, SO₃H, SO₃Na ou un groupe aryle en C₆-C₁₂ qui est non substitué ou substitué par un ou plusieurs atomes d'halogène, un ou plusieurs groupes alkyle en C₁-C₆ linéaires ou ramifiés, nitro, acétyle ou SO₂NHC₁-C₆alkyle ou par SO₂NH₂.

Les compositions cosmétiques conformes à l'invention renferment au moins un pigment latent et au moins un composé choisi parmi les agents acidifiants, les agents tensioactifs, les monoalcools ou les polyols liquides à 25°C.

Les agents acidifiants des compositions de l'invention peuvent être minéraux ou organiques. Dans ce dernier cas, les fonctions apportant l'acidité peuvent être des groupements sulfoniques ou carboxyliques. On peut citer comme agents acidifiants utilisables selon l'invention, l'acide chlorhydrique, l'acide citrique, l'acide lactique, l'acide tartrique.

La concentration en agents acidifiants peut varier de 0,0001% à 20%, de préférence de 0,01% à 10% du poids total des compositions.

Les monoalcools ou les polyols liquides à 25°C des compositions selon l'invention sont saturés ou non. Ils sont de préférence choisis parmi l'éthanol, l'isopropanol, le propylènegycol, le glycérol, l'héxylèneglycol, l'isoprèneglycol, le dipropylèneglycol, le néopentylglycol, le 3-méthyl-1,3,5-pentanetriol, le 1,2,4-butanediol, le 1,5-pentanediol, le 2-méthyl-1,3-propanediol, le 3-méthyl-1,5-pentanediol, les polyéthylèneglycols, l'alcool benzylique.

Leur concentration peut être comprise entre 0,05% et 50% et de préférence entre 0,1% et 20% du poids total de la composition.

Les tensioactifs peuvent être de nature anionique, non ionique, cationique ou amphotère. Les tensioactifs convenant à la mise en oeuvre de la présente invention sont notamment les suivants :

### (i) Tensioactif(s) anionique(s) :

A titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkyl(C₆-C₂₄) sulfosuccinates, les alkyl(C₆-C₂₄) éthersulfosuccinates, les alkyl(C₆-C₂₄) amidesulfosuccinates ; les alkyl(C₆-C₂₄) sulfoacétates ; les acyl(C₆-C₂₄) sarcosinates et les acyl(C₆-C₂₄) glutamates. On peut également utiliser les esters d'alkyl(C₆-C₂₄)polyglycosides carboxyliques tels que les alkylglucoside citrates, les alkylpolyglycoside tartrate et les alkylpolyglycoside sulfosuccinates., les alkylsulfosuccinamates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser les acides d'alkyl D galactoside uroniques et leurs sels, les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'alkylène en particulier d'éthylène, et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alphadiols, les alkylphénols polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensio-actifs non-ioniques rentrant particulièrement bien dans le cadre de la présente invention.

### (iii) Tensioactif(s) amphotère(s) ou zwittérionique(s) :

Les agents tensioactifs amphotères ou zwitterioniques, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

R₂-CONHCH₂CH₂-N(R₃)(R₄)(CH₂COO⁻)

dans laquelle : R₂ désigne un radical alkyle linéaire ou ramifié en C₅-C₂₀ provenant d'un acide R₂-COOH présent par exemple dans l'huile de coprah hydrolysée, un radical heptyle, nonyle ou undécyle, R₃ désigne un groupement bêta-hydroxyéthyle et R₄ un groupement carboxyméthyle ; et

R₂'-CONHCH₂CH₂-N(B)(C)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂- CHOH - SO₃H
R₂' désigne un radical alkyle linéaire ou ramifié, saturé ou non, en C₅-C₂₀ d'un acide R'₂-COOH présent par exemple dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL® C2M concentré par la société RHODIA CHIMIE.

### (iv) Tensioactifs cationiques :

Parmi les tensioactifs cationiques on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

Leur concentration peut être comprise entre 0,05% et 50% et de préférence entre 0,2 et 25% du poids total de la composition.

Le milieu approprié pour la teinture appelé aussi support de teinture est de préférence un milieu aqueux contenant au moins un des ingrédients ci-dessus.

De préférence, les compositions selon l'invention ont un pH inférieur à 7.

On peut utiliser dans les compositions des adjuvants cosmétiques classiques utilisés dans les compositions tinctoriales tels que des agents antioxydants, des agents de pénétration, des agents séquestrants, des parfums, des tampons, des agents dispersants, des agents filmogènes, des céramides, des agents conservateurs, des agents opacifiants, des vitamines ou provitamines, des polymères non ioniques, cationiques, anioniques ou amphotères, des agents épaississants minéraux ou organiques, associatifs ou non.

Bien entendu, l'homme de l'art veillera à choisir ce ou ces éventuels composés complémentaires de manière telle que les propriétés avantageuses attachées intrinsèquement à la composition de teinture conforme à l'invention ne soient pas, ou substantiellement pas, altérées par la ou les adjonctions envisagées.

Un autre objet de l'invention concerne un procédé de coloration de fibres kératiniques en particulier de fibres kératiniques humaines et plus particulièrement les cheveux mettant en oeuvre les pigments latents.

Dans un premier temps, on applique sur les fibres kératiniques une composition contenant dans un milieu approprié pour la teinture au moins un pigment latent utilisé selon l'invention.

On laisse agir la composition colorante pendant un temps de pose variant de 1 à 60 minutes, et de préférence de 10 à 45 minutes.

Les fibres sont alors rincées ou non.

On traite ensuite le pigment latent sur lesdites fibres par voie thermique, chimique ou photochimique afin de régénérer le composé d'origine peu soluble. De préférence, ce dernier traitement est réalisé par un saut de pH. La solution utilisée permet d'atteindre un pH supérieur à 7. Une solution d'ammoniaque, d'alcanolamine, d'un hydroxyde alcalin ou d'un carbonate alcalin peut être utilisée.

Enfin, on effectue des étapes de lavage au shampooing et de séchage.

L'invention concerne également un dispositif à 2 compartiments pour la teinture des fibres. Le premier compartiment de ce dispositif renferme une composition colorante contenant, dans un milieu approprié pour la teinture, au moins un pigment latent utilisé selon l'invention, le second compartiment renferme une solution d'agent chimique susceptible de rendre insoluble le pigment latent en composé d'origine peu soluble. Le second compartiment renfermera de préférence une solution permettant d'atteindre un pH supérieur à 7.

L'exemple suivant est destiné à illustrer l'invention sans présenter un caractère limitatif.

### Exemple :

La demanderesse a réalisé une composition contenant le composé A représenté ci-dessous.

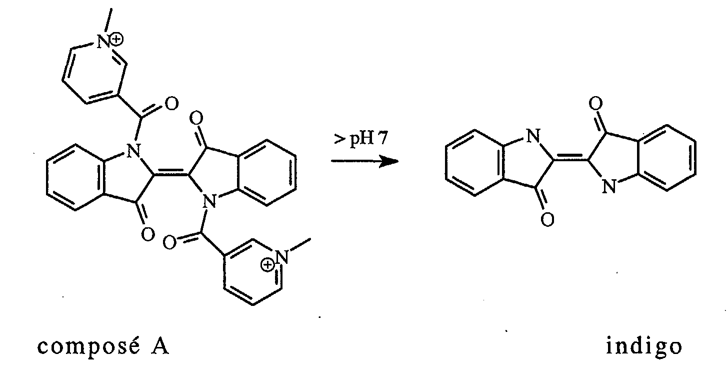

La composition contenant le composé A a été formulée comme suit :

| Formule de coloration | |
|---|---|
| Natrosol 250MR (hydroxyéthyl cellulose) | 0,72g |
| NIPA ester 82121 (mélange p-hydroxybenzoates de méthyle, butyle, éthyle, propyle, isobutyle) | 0,06g |
| ORAMIX CG 110 (alkyl (C8/C10 50/50) polyglucoside (2) en solution aqueuse à 60% tamponnée) | 5g |
| Alcool benzylique | 4g |
| Polyéthylèneglycol 400(polyéthylène plycol (8 OE)) | 6g |
| Composé A | 0,3g |
| Acide citrique | QSP |
| eau | pH 4 QSP |

La composition a été appliquée sur des cheveux faiblement décolorés avec un rapport de bain de 10.

Après un temps de pose de 30 minutes, on applique sur la mèche une solution diluée de triéthanolamine. Ainsi la réaction de régénération a été réalisée par un saut de pH. La couleur de la mèche passe alors du violet au bleu.

La mèche est ensuite shampooinée et séchée. Elle est bleue et la coloration présente un très bon niveau de ténacité.

## Revendications

1. Utilisation pour la coloration de fibres kératiniques, en particulier de fibres kératiniques humaines, plus particulièrement les cheveux, d'au moins un pigment latent, soluble dans un milieu approprié pour la teinture, susceptible d'être transformé dans les fibres en pigment insoluble dans l'eau par voie chimique, thermique ou photochimique.

2. Utilisation selon la revendication 1 dans laquelle le pigment latent utilisé selon la présente invention est représenté par la formule (I)
A(B)ₓ (I)
dans laquelle x représente un nombre entier allant de 1 à 8,
A représente le radical chromophore de colorants comprenant un hétéroatome choisi parmi N, O et S, et
• lorsque x=1, B représente un groupe de formule (II),
• lorsque x est supérieur à 1, B désigne un atome d'hydrogène ou un groupe de formule (II),
B désignant au moins une fois un groupe de formule (II),
Le groupe de formule (II) correspond à 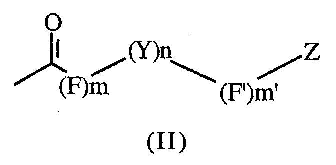 dans laquelle
Z représente un groupement hydrosolubilisant cationique Z⁺ ou un résidu de polyéthylèneglycol,
Y représente un hétéroatome choisi dans le groupe formé par N, O, et S, Y étant de préférence O,
F et F' représentent, indépendamment l'un de l'autre, une chaîne alkylène en C₁-C₁₄ linéaire ou ramifiée, pouvant contenir des hétéroatomes et pouvant être substituée par un ou plusieurs groupements hydroxy, amino, halogène,
n, m, m' désignent indépendamment les uns des autres, 0 ou 1,
B étant lié à un hétéroatome choisi dans le groupe N, O, et S du chromophore A.

3. Utilisation selon la revendication 2, dans laquelle Z⁺ est un groupe aliphatique, un groupe aromatique, un groupe carbocyclique saturé ou non, un groupe hétérocyclique et porte au moins un atome d'azote quaternisé.

4. Utilisation selon les revendications 1 et 2, dans laquelle le chromophore A est le radical de colorants de type pérylène, quinacridone, dioxazine, isoindoline, indigo, bisisoindoline, phtalocyanine, pyrrolo-pyrrole, quinophtalone, azo, anthraquinone, indanthrone, isoindolinone, naphtoquinone, benzoquinone, azométhine.

5. Utilisation selon les revendications 1 à 3, dans lesquelles le radical chromophore du colorant A est choisi parmi :
- les dérivés du pérylène de formules (III) ou (IV) 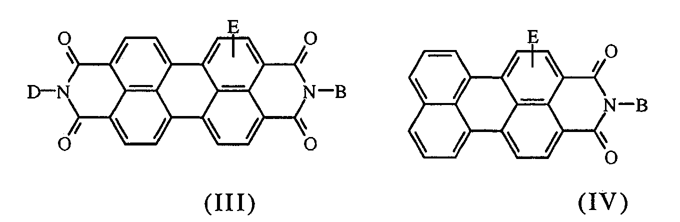 dans lesquelles D représente un atome d'hydrogène, un atome d'halogène, un groupe alkyle en C₁-C₂₄ linéaire ou ramifié, de préférence en C₁-C₆ ou un groupe phényle, benzyle, phénéthyle substitués ou non par un groupe alkyle en C₁-C₆, ou un groupe de formule B,
E représente un atome d'hydrogène, un atome d'halogène, un groupe alkyle en C₁-C₂₄ linéaire ou ramifié, de préférence en C₁-C₆, un groupe alcoxy en C₁-C₆ ou un groupe phényle,
- les quinacridones de formule (V) 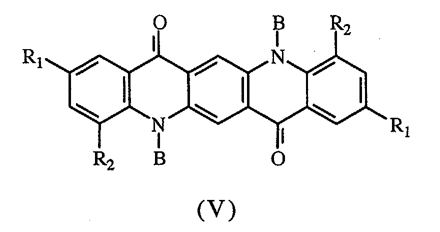 dans laquelle R₁ et R₂, indépendamment l'un de l'autre, représentent un atome d'hydrogène, un atome d'halogène, un groupe alkyle en C₁-C₂₄ linéaire ou ramifié, de préférence en C₁-C₆, un groupe alcoxy en C₁-C₆ ou un groupe phényle,
- des dioxazines de formules (VI) ou (VII) 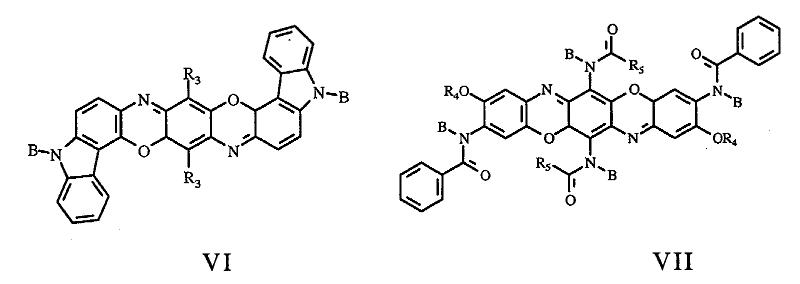 dans lesquelles R₃ représente un atome d'hydrogène, un atome d'halogène, ou un groupe alkyle en C₁-C₂₄ linéaire ou ramifié, de préférence en C₁-C₆,
R₄ et R₅, indépendamment l'un de l'autre, représentent un groupe alkyle en C₁-C₄,
- les isoindolines de formules (VIII), (IX) ou (X) 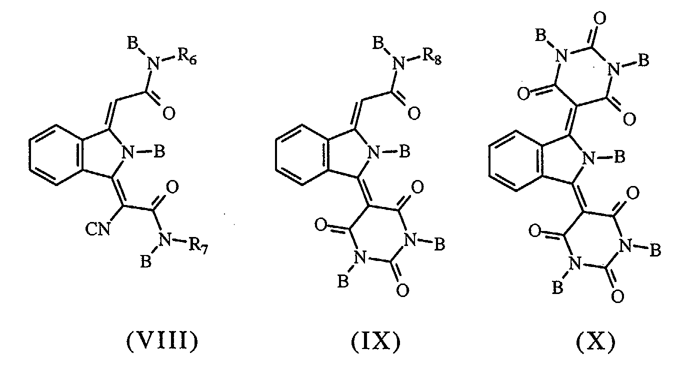 dans lesquelles R₆ est représenté par la formule (XI),
R₇ représente un atome d'hydrogène, un groupe alkyle en C₁-C₂₄ linéaire ou ramifié, de préférence en C₁-C₆, un groupe benzyle ou un groupe de formule (XII),
R₈ représente un atome d'hydrogène, le groupe de formule (XI) ou le groupe B, 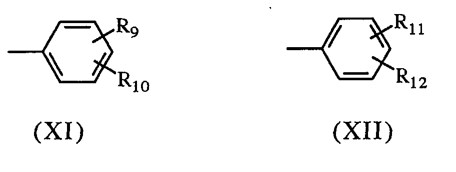 R₉, R₁₀, R₁₁,R₁₂, indépendamment les uns des autres, représentent un atome d'hydrogène, un atome d'halogène, un groupe alkyle en C₁-C₂₄ linéaire ou ramifié, de préférence en C₁-C₆, un groupe alkoxy en C₁-C₆ ou un groupe trifluorométhyle,
- les dérivés d'indigo de formule (XIII) 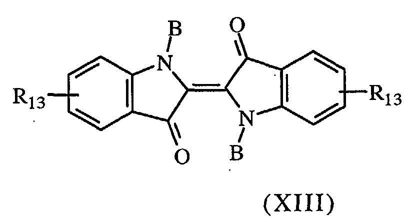 dans laquelle R₁₃ représente un atome d'hydrogène, un atome d'halogène, un groupe alkyle en C₁-C₆ linéaire ou ramifié, un groupe alkoxy en C₁-C₆ ou un groupe nitrile.
- les dérivés de bisindolinones de formules (XIV) ou (XV) 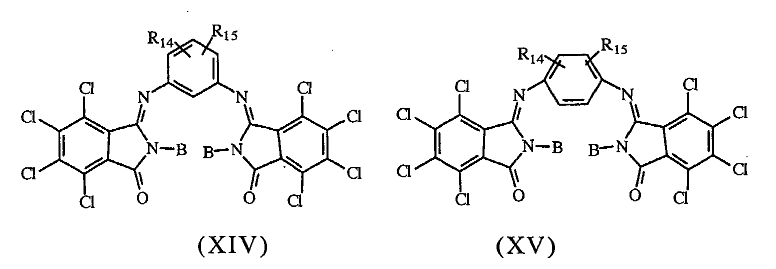 dans lesquelles R₁₄ et R₁₅, indépendamment l'un de l'autre, représentent un atome d'hydrogène, un atome d'halogène, un groupe alkyle en C₁-C₄ linéaire ou ramifié,
- les dérivés d'anthraquinoïdes de formules (XVI) ou (XVII) 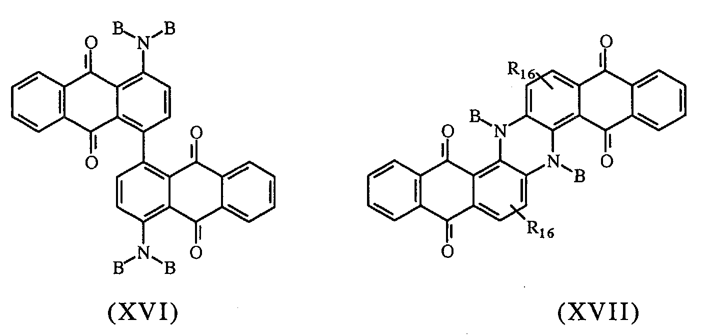 dans lesquelles R₁₆ représente un atome d'hydrogène ou un atome d'halogène.
- les dérivés de phthalocyanine de formule (XVIII) 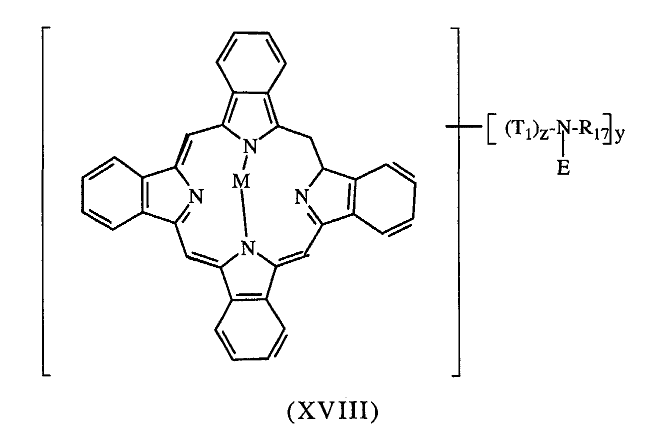 dans laquelle M représente H₂, un métal divalent choisi parmi le cuivre, le magnésium, le fer, le zinc, l'aluminium, le manganèse, le calcium, le baryum ou un groupe métallique divalent tel que MnO ou TiO,
T₁ représente le groupe -CHR₁₈-, -CO-, -SO₂-,
R₁₇ représente un atome d'hydrogène, un groupe alkyle en C₁-C₆ linéaire ou ramifié, un groupe -N(B)R₁₈, -N(B)₂, -NHCOR₁₉, -COR₁₉, ou le groupe de formule 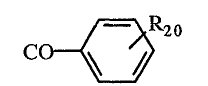 R₁₈ représente un atome d'hydrogène, un groupe alkyle en C₁-C₆ linéaire ou ramifié,
R₁₉ représente un groupe alkyle en C₁-C₆ linéaire ou ramifié,
R₂₀ représente un atome d'hydrogène, un atome d'halogène, un groupe alkyle en C₁-C₆ linéaire ou ramifié, un groupe alkoxy en C₁-C₆,
z représente 0 ou 1,
y représente un nombre entier de 1 à 8,
- les dérivés de pyrrolo-pyrroles de formules (XIX) ou (XX) 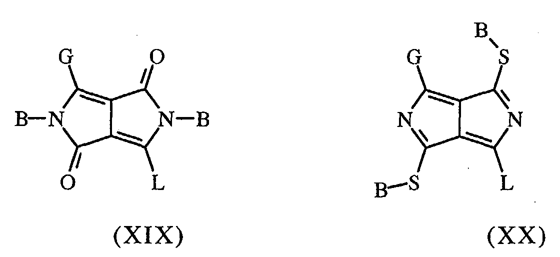 dans lesquels G et L, indépendamment l'un de l'autre, peuvent avoir comme signification : 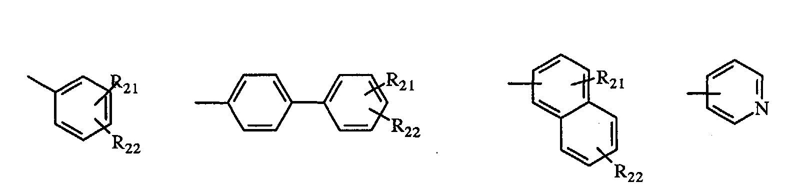 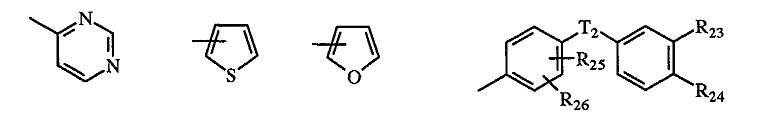 R₂₁ et R₂₂, indépendamment l'un de l'autre, représentent un atome d'hydrogène, un atome d'halogène, un groupe alkyle en C₁-C₂₄ linéaire ou ramifié, de préférence en C₁-C₆, un groupe alkoxy en C₁-C₆, un groupe alkylthio en C₁-C₁₈, un groupe alkylamino en C₁-C₁₈, un groupe cyano, nitro, phényle, trifluorométhyle, cycloalkyle en C₅-C₆, un groupe -C=N-(alkyle C₁-C₂₄, de préférence en C₁-C₆), un groupe de formule 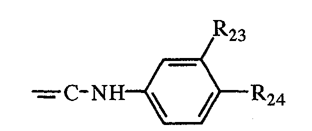 un groupe imidazolyle, pyrazolyle, triazolyle, pipérazinyle, pyrrolyle, oxazolyle, benzoxazolyle, benzothiazolyle, benzimidazolyle, morpholinyle, pipéridinyle ou pyrrolidinyle,
T₂ représente -CH₂-, -CH(CH₃)-, -C(CH₃)₂-, -CH=N-, -N=N-, -O-, -S-, -SO-, -SO₂- ou -NHR₂₇,
R₂₃ et R₂₄, indépendamment l'un de l'autre, représentent un atome d'hydrogène, un halogène, un groupe alkyle en C₁-C₆ linéaire ou ramifié, un groupe alkoxy en C₁-C₆ ou -CN,
R₂₅ et R₂₆, indépendamment l'un de l'autre, représentent un atome d'hydrogène, un atome d'halogène, un groupe alkyle en C₁-C₆ linéaire ou ramifié,
R₂₇ représente un atome d'hydrogène ou un groupe alkyle en C₁-C₆ linéaire ou ramifié,
- les dérivés de quinophthalones de formules (XXI) ou (XXII) 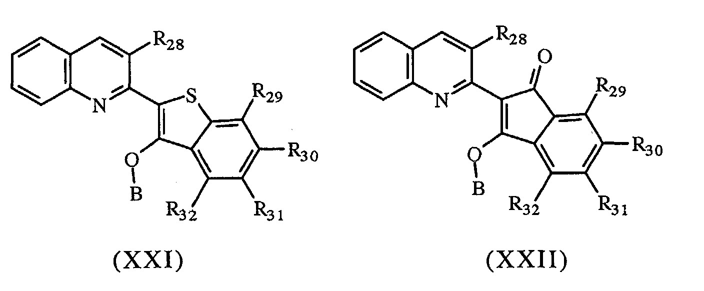 dans lesquelles R₂₈ représente un atome d'hydrogène ou un groupe OB,
R₂₉, R₃₀, R₃₁ et R₃₂, indépendamment les uns des autres, représentent un atome d'hydrogène, un atome d'halogène, un groupe COOC₁-C₆alkyle linéaire ou ramifié, ou un groupe CONHC₁-C₆alkyle linéaire ou ramifié,
- les composés azoïques de formules de (XXIII) à (XXVIII) 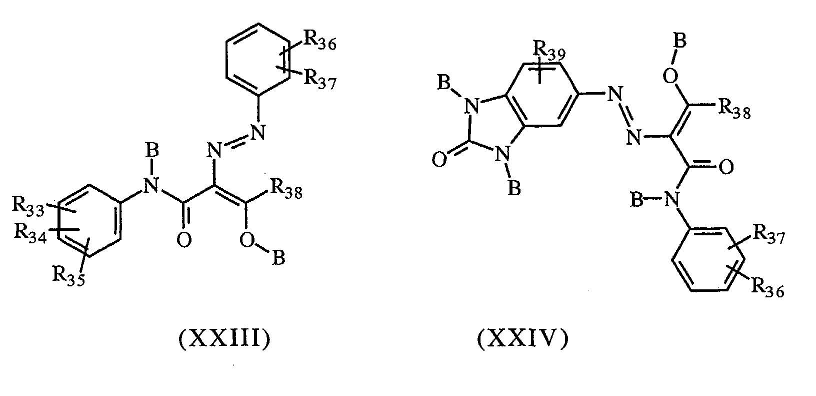 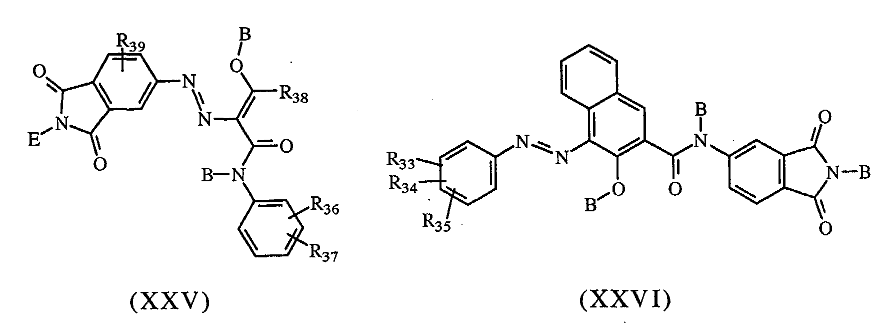 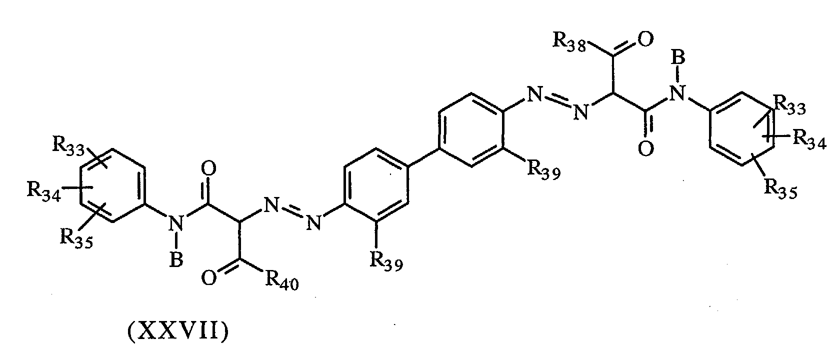 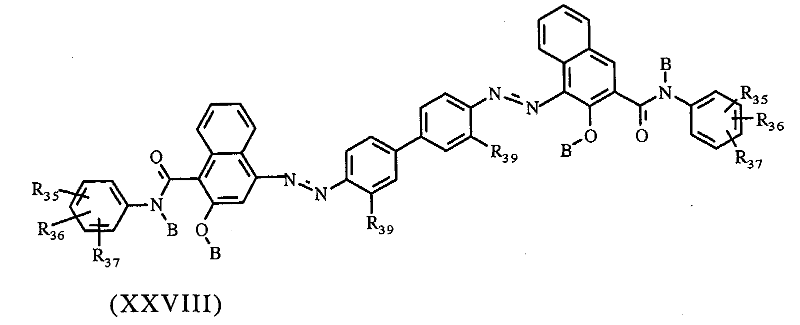 dans lesquelles R₃₃, R₃₄, R₃₅, R₃₆, R₃₇, R₃₈ et R₄₀ indépendamment les uns des autres, représentent chacun un atome d'hydrogène, un atome d'halogène, un groupe alkyle en C₁-C₆ linéaire ou ramifié, un groupe alkoxy en C₁-C₆ , un groupe nitro, acétyle ou un groupe SO₂NHC₁-C₆alkyle,
R₃₉ représente un atome d'hydrogène, un atome d'halogène, un groupe alkyle en C₁-C₆ linéaire ou ramifié, un groupe alkoxy en C₁-C₆ ,
- les dérivés d'anthraquinones de formule (XXIX) ou (XXX) 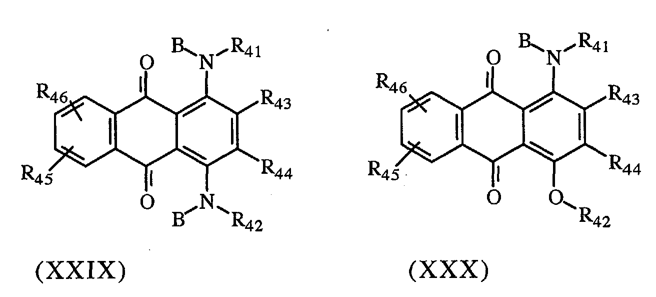 dans lesquelles R₄₁ et R₄₂, indépendamment l'un de l'autre, représentent un atome d'hydrogène, un atome d'halogène, un groupe alkyle en C₁-C₁₂ linéaire ou ramifié, un groupe alkoxy en C₁-C₆, ou un groupe aryle en C₆-C₁₂ qui est non substitué ou substitué par un ou plusieurs atomes d'halogène, un ou plusieurs groupes alkyle en C₁-C₆ linéaires ou ramifiés, nitro, acétyle ou groupe SO₂NHC₁-C₆alkyle ou SO₂NH₂,
R₄₃ et R₄₄, indépendamment l'un de l'autre, représentent un atome d'hydrogène, un atome d'halogène, un groupe alkyle en C₁-C₆ linéaire ou ramifié, un groupe alkoxy en C₁-C₆, un groupe nitro, cyano, CONH₂, SO₂NHC₁-C₆ alkyle, SO₂NH₂, SO₃H, SO₃Na ou un groupe aryle en C₆-C₁₂ qui est non substitué ou substitué par un ou plusieurs atomes d'halogène, un ou plusieurs groupes alkyle en C₁-C₆ linéaires ou ramifiés, nitro, acétyle ou SO₂NHC₁-C₆alkyle ou par SO₂NH₂,
R₄₅ et R₄₆ représentent indépendamment l'un de l'autre, un atome d'hydrogène, un atome d'halogène, un groupe alkoxy en C₁-C₆, un groupe nitro, cyano, hydroxyle ou amino,
- les dérivés d'azométhine de formule (XXXI) 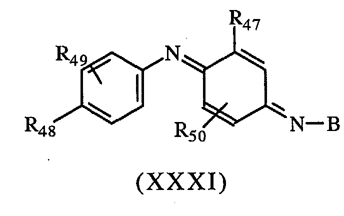 R₄₇, R₄₈, R₄₉ et R₅₀, indépendamment les uns des autres, représentent un atome d'hydrogène, un atome d'halogène, un groupe alkyle en C₁-C₆ linéaire ou ramifié, un groupe alkoxy en C₁-C₆ , un groupe nitro, cyano, CONH₂, SO₂NHC₁-C₆ alkyle, SO₂NH₂, SO₃H, SO₃Na ou un groupe aryle en C₆-C₁₂ qui est non substitué ou substitué par un ou plusieurs atomes d'halogène, un ou plusieurs groupes alkyle en C₁-C₆ linéaires ou ramifiés, nitro, acétyle ou SO₂NHC₁-C₆alkyle ou par SO₂NH₂.

6. Procédé de coloration de fibres kératiniques en particulier de fibres kératiniques humaines et plus particulièrement les cheveux, ce procédé comprenant les étapes suivantes :
(i) on applique sur les fibres kératiniques une composition contenant dans un milieu approprié pour la teinture, un ou plusieurs pigments latents selon les revendications 1 à 5,
(ii) on laisse agir la composition colorante pendant un temps de pose variant de 1 à 60 minutes, et de préférence de 10 à 45 minutes,
(iii) après un rinçage éventuel, on traite ensuite le pigment latent sur lesdites fibres par voie thermique, chimique ou photochimique, de préférence par un saut de pH,
(iv) on effectue des étapes de lavage au shampooing et de séchage.

7. Procédé selon la revendication 6, **caractérisé par le fait que** l'on traite le pigment latent sur les fibres kératiniques par une solution d'un composé alcalin.

8. Procédé selon la revendication 7, **caractérisé par le fait que** l'agent alcalin est choisi parmi l'ammoniaque, les alcanolamines, les hydroxydes alcalins, les carbonates alcalins.

9. Composition cosmétique contenant au moins un pigment latent et au moins un composé choisi parmi les monoalcools ou les polyols liquides à 25°C, les agents acidifiants, les agents tensioactifs.

10. Composition selon la revendication 9, **caractérisée par le fait que** le pigment latent est de formule (I) définie à l'une des revendications 1 à 5.

11. Composition selon la revendication 9 ou 10, **caractérisée par le fait que** l'agent acidifiant est de nature minérale ou organique.

12. Composition selon l'une quelconque des revendications 9 à 11, **caractérisée par le fait que** le tensioactif est de nature non ionique, anionique, cationique ou amphotère.

13. Composition selon l'une quelconque des revendications 9 à 12, **caractérisée par le fait que** la concentration en agent acidifiant est comprise entre 0,0001% et 20% et de préférence entre 0,01% et 10% du poids total de la composition.

14. Composition selon l'une quelconque des revendications 9 à 13, **caractérisée par le fait que** la concentration en agent(s) tensioactif(s) est comprise entre 0,05% et 50% et de préférence entre 0,1% et 20% du poids total de la composition.

15. Composition selon l'une des revendications 9 à 14, **caractérisée par le fait que** la concentration en monoalcools ou polyols liquides à 25°C est comprise entre 0,05% et 50%, de préférence entre 0,1% et 20% du poids total de la composition.

16. Composition selon l'une quelconque des revendications 9 à 15, **caractérisée par le fait que** son pH est inférieur à 7.

17. Dispositif à 2 compartiments pour la teinture des fibres kératiniques en particulier de fibres kératiniques humaines et plus particulièrement les cheveux, **caractérisé par le fait qu'**un premier compartiment renferme une composition colorante contenant, dans un milieu approprié pour la teinture, au moins un pigment latent utilisé selon les revendications 1 à 5, un second compartiment renferme une solution diluée d'un agent chimique susceptible de rendre insoluble le pigment latent en composé d'origine peu soluble.

18. Dispositif selon la revendication 17, **caractérisé par le fait que** le second compartiment renferme une solution d'un agent alcalin.

19. Dispositif selon la revendication 17, **caractérisé par le fait que** le premier compartiment renferme une composition selon l'une quelconque des revendications 9 à 16.
